# EUROPEAN PATENT APPLICATION

(11) **EP 3 153 209 A1**
(43) Date of publication of application: **12.04.2017**
(21) Application number: 15803605.3
(22) Date of filing: 27.05.2015
(51) Int. Cl.: A61N 1/04

(54) **REHABILITATION ASSISTANCE SYSTEM**

(30) Priority: 04.06.2014 JP 2014115858
(71) Applicant: Nihon Kohden Corporation, Shinjyuku-ku Tokyo 161-8560 (JP)
(72) Inventor: ONO Yoshinobu, Tokyo 161-8560 (JP); MATSUMOTO Hiroyuki, Tokyo 161-8560 (JP); YADE Masahiro, Tokyo 161-8560 (JP); MINEGISHI Yuka, Tokyo 161-8560 (JP); SUZUKI Takehito, Tokyo 161-8560 (JP)
(74) Representative: Hoffmann Eitle
(86) International application number: PCT/JP2015/065282
(87) International publication number: WO 2015/186587

(57) **Abstract**

A stimulation electrode (2) is to be placed on a paralyzed limb (100) of a patient. A placement position storing section (4) stores placement position information indicative of a position where the stimulation electrode (2) is to be placed. A guidance information providing section (4) provides guidance information for guiding the stimulation electrode (2) to the position based on the placement position information, to a user.

## Description

### Technical Field

The present invention relates to a system for assisting rehabilitation which is performed on a hemiplegia patient after, for example, stroke.

### Background Art

In this kind of rehabilitation, a stimulation electrode is placed on the paralyzed limb of the patient. Based on instructions from the brain to try to move the paralyzed limb, the stimulation electrode is energized, and the paralyzed limb is electrically stimulated. This can cause the patient to feel as if the paralyzed limb moves as intended (for example, see Patent Literature 1).

### Citation List

### Patent Literature

Patent Literature 1: JP-A-2009-112791

### Summary of Invention

### Technical Problem

In order to enable an effect of rehabilitation which is performed in a plurality of opportunities, to be properly exerted, and to adequately evaluate the effect, a position where a stimulation electrode is placed on the patient is required to be constant.

Therefore, it is an object of the invention that, in a plurality of opportunities, a position where a stimulation electrode is placed on the patient is made constant irrespective of the practitioner.

In order to attain the object, a first mode which the invention may have is a rehabilitation assistance system comprising:
a stimulation electrode which is to be placed on a part of a body of a patient;
a placement position storing section which stores placement position information indicative of a position where the stimulation electrode is to be placed; and
a guidance information providing section which, based on the placement position information, provides guidance information for guiding the stimulation electrode to the position, to a user.

Based on the guidance information provided by the guidance information providing section, the practitioner (an example of the user) places the stimulation electrode on a predetermined position on the part of the body of the patient. According to the configuration, in a plurality of opportunities, a position where the stimulation electrode is placed on the patient is made constant irrespective of the practitioner. The position is determined as a position where an effect of rehabilitation is properly exerted. Therefore, the effectiveness of rehabilitation which is performed in a plurality of opportunities can be enhanced. Moreover, the placement position of the stimulation electrode is always constant, and therefore the reliability of evaluation of the effect of rehabilitation which is performed in a plurality of opportunities can be enhanced.

The guidance information providing section may optically indicate the guiding information on the part of the body of the patient.

According to the configuration, the practitioner can correctly place the stimulation electrode at a predetermined position while being visually guided by the guidance information which is optically indicated. In a plurality of opportunities, therefore, the position where the stimulation electrode is placed on the patient can be made constant irrespective of the practitioner.

The rehabilitation assistance system may further comprise a display apparatus, and the guidance information providing section may cause the guidance information to be displayed on the display apparatus.

As an example, a case where the guidance information contains an image of the part of the body of the patient, which is acquired in a past will be considered. In this case, the practitioner can correctly place the stimulation electrode at a predetermined position while checking the position where the stimulation electrode was placed in past rehabilitation. In a plurality of opportunities, therefore, the position where the stimulation electrode is placed on the patient can be made constant irrespective of the practitioner.

As another example, a case where the guidance information is a numerical value indicating a distance to the placement position will be considered. In this case, the practitioner can correctly place the stimulation electrode at a predetermined position while checking that the numerical value approaches zero. In a plurality of opportunities, therefore, the position where the stimulation electrode is placed on the patient can be made constant irrespective of the practitioner.

The guidance information may be a sound which changes in accordance with a distance to the placement position.

According to the configuration, the practitioner can correctly place the stimulation electrode at a predetermined position while being acoustically guided by the output sound. In a plurality of opportunities, therefore, the position where the stimulation electrode is placed on the patient can be made constant irrespective of the practitioner.

The rehabilitation assistance system may further comprise:
an image acquiring section which acquires an image of the part of the body of the patient; and
an electrode position information acquiring section which, from the image, acquires electrode position information indicative of a position where the stimulation electrode is placed on the patient.

In this case, the placement position storing section stores the stimulation electrode position information as the placement position information.

According to the configuration, the placement of the stimulation electrode which is determined in rehabilitation performed at a certain timing is stored, and can be used in the next and subsequent rehabilitation processes. In a plurality of opportunities, therefore, the position where the stimulation electrode is placed on the patient can be made constant irrespective of the practitioner.

The rehabilitation assistance system may further comprise
a light emitter which is placed adjacent to the stimulation electrode, and which emits light having a predetermined wavelength.

According to the configuration, the position of the stimulation electrode is detected more easily by an image recognition technique performed by the image acquiring section. Consequently, the position of the stimulation electrode is detected more correctly, and the accuracy of the placement position information which is to be stored in the placement position storing section can be improved. In a plurality of opportunities, therefore, the position where the stimulation electrode is placed on the patient can be made constant irrespective of the practitioner.

The rehabilitation assistance system may further comprise
a normalized scale which is to be placed adjacent to the part of the body of the patient.

According to the configuration, the image acquiring section performs image recognition with respect to the scale, and can correctly specify a position on the part of the body of the patient without using the distance between the image acquiring section and the part of the body of the patient, or the like. Consequently, the position of the stimulation electrode is detected more correctly, and the accuracy of the placement position information which is to be stored in the placement position storing section can be improved. In a plurality of opportunities, therefore, the position where the stimulation electrode is placed on the patient can be made constant irrespective of the practitioner.

The stimulation electrode position information may contain information indicative of a feature point included in the part of the body of the patient.

According to the configuration, even when a scale or the like is not used, the position of the stimulation electrode can be specified by using the feature point on the part of the body of the patient. The feature point itself is not changed over a plurality of processes of rehabilitation, and hence the placement position of the stimulation electrode can be specified with respect to the feature point also in the next and subsequent rehabilitation processes. In a plurality of opportunities, therefore, the position where the stimulation electrode is placed on the patient can be made constant irrespective of the practitioner.

The rehabilitation assistance system may further comprise:
a stimulation applying section which applies electrical stimulation to the part of the body of the patient through the stimulation electrode; and
an evaluating section which evaluates an effect of rehabilitation based on a change of the stimulation electrode position information, which is caused by the electrical stimulation.

According to the configuration, a reaction of the part of the body of the patient to electrical stimulation, i.e., the effect of rehabilitation can be quantitatively evaluated without depending on visual observation by an observer. Electrical stimulation is performed through the stimulation electrode which is correctly placed at the constant position as described above. In a plurality of opportunities, therefore, correctness can be ensured in both an input of stimulation and a reaction to the stimulation.

The rehabilitation assistance system may further comprise
an inertial sensor which is placed adjacent to the stimulation electrode.

In this case, the evaluating section evaluates the effect of rehabilitation based on an output of the inertial sensor.

According to the configuration, it is possible to acquire information of not only displacement of the stimulation electrode, but also the displacement velocity and attitude change of the stimulation electrode. Therefore, the effect of rehabilitation can be evaluated in more detail.

The rehabilitation assistance system may further comprise
at least one of a temperature sensor and a moisture sensor which are placed adjacent to the stimulation electrode.

In this case, the evaluating section evaluates the effect of rehabilitation based on an output of the at least one of the temperature sensor and the moisture sensor.

It is known that the more advancement of recovery of the function of a paralyzed portion, the higher temperature and moisture of the portion are observed. According to the configuration, it is possible to acquire information of the degree of recovery based on not only displacement of the stimulation electrode, but also at least one of the temperature and moisture of a part of the body of the patient. Therefore, the effect of rehabilitation can be evaluated in more detail.

The rehabilitation assistance system may further comprise
a recording section which records the stimulation electrode position information while associating the stimulation electrode position information with at least a timing when the electrode position information is acquired, and patient identification information.

In this case, the evaluating section compares the stimulation electrode position information at a plurality of timings with one another to evaluate the effect of rehabilitation.

According to the configuration, a change of a reaction of the part of the body of the patient to the electrical stimulation, i.e., the progress of rehabilitation can be quantitatively evaluated without depending on visual observation by an observer. Electrical stimulation is performed through the stimulation electrode which is correctly placed at the constant position as described above. In a plurality of opportunities, therefore, correctness can be ensured in both an input of stimulation and a reaction to the stimulation irrespective of the practitioner.

In order to attain the object, a second mode which the invention may have is a rehabilitation assistance system comprising:
a stimulation applying section which applies electrical stimulation to a part of a body of a patient;
an image acquiring section which acquires an image of the part of the body of the patient;
an evaluating section which evaluates a degree of a reaction of the part of the body of the patient to the electrical stimulation, based on the image;
a displaying section which displays the image; and
a display controller which causes a map indicating relationships between a position where the electrical stimulation is applied, and the degree of the reaction, to be displayed on the displaying section.

According to the configuration, irrespective of an evaluator, the placement position of the stimulation electrode can be determined based on a quantitative evaluation. When the stimulation electrode is placed at the placement position which is determined as described above, the position where the stimulation electrode is placed on the patient can be made constant irrespective of the practitioner in a plurality of opportunities.

The stimulation applying section may include a conducting portion which is disposed in a tip end portion of a rod member.

According to the configuration, without conducting a work of attaching/detaching the stimulation electrode to and from a part of the body of the patient, the application of electrical stimulation to a plurality of arbitrary places on the part of the body can be efficiently performed. Therefore, a quantitative evaluation for determining the placement position of the stimulation electrode can be efficiently executed.

The stimulation applying section may include a plurality of conducting portions which are arranged on a sheet member to be attached to the part of the body of the patient.

According to the configuration, when the sheet member is once attached to a part of the body of the patient, the application of electrical stimulation to a plurality of places on the part of the body can be efficiently performed. Particularly, the positional relationships among the plurality of conducting portions are fixed, and hence it is possible to eliminate a situation where the stimulation position is differentiated depending on the practitioner. Therefore, a quantitative evaluation for determining the placement position of the stimulation electrode can be efficiently executed irrespective of the practitioner.

The display controller may cause an image indicating a position of at least one of muscle and nerve which are included in the part of the body of the patient, to be displayed on the displaying section.

According to the configuration, the application position of stimulation performed by the stimulation applying section can be determined by reference to the image. Therefore, application of electrical stimulation to a plurality of places on the part of the body can be efficiently performed.

### Brief Description of Drawings

[Fig. 1] Fig. 1 is a diagram illustrating a rehabilitation assistance system of a first embodiment.
[Fig. 2] Fig. 2 is a view illustrating stimulation electrodes provided in the system.
[Fig. 3] Figs. 3(a) and 3(b) are views illustrating an example of a guidance information providing section provided in the system.
[Fig. 4] Figs. 4(a) and 4(b) are views illustrating an example of the guidance information providing section provided in the system.
[Fig. 5] Figs. 5(a) and 5(b) are views illustrating an example of the guidance information providing section provided in the system.
[Fig. 6] Fig. 6 is a view illustrating an example of the guidance information providing section provided in the system.
[Fig. 7] Figs. 7(a) and 7(b) are views illustrating an example of a configuration for acquiring position information of the stimulation electrode.
[Fig. 8] Fig. 8 is a view illustrating an example of a configuration for evaluating an effect of rehabilitation.
[Fig. 9] Fig. 9 is a diagram illustrating a rehabilitation assistance system of a second embodiment.
[Fig. 10] Figs. 10(a) and 10(b) are views illustrating an example of a stimulation applying section provided in the system of Fig. 9.

### Description of Embodiments

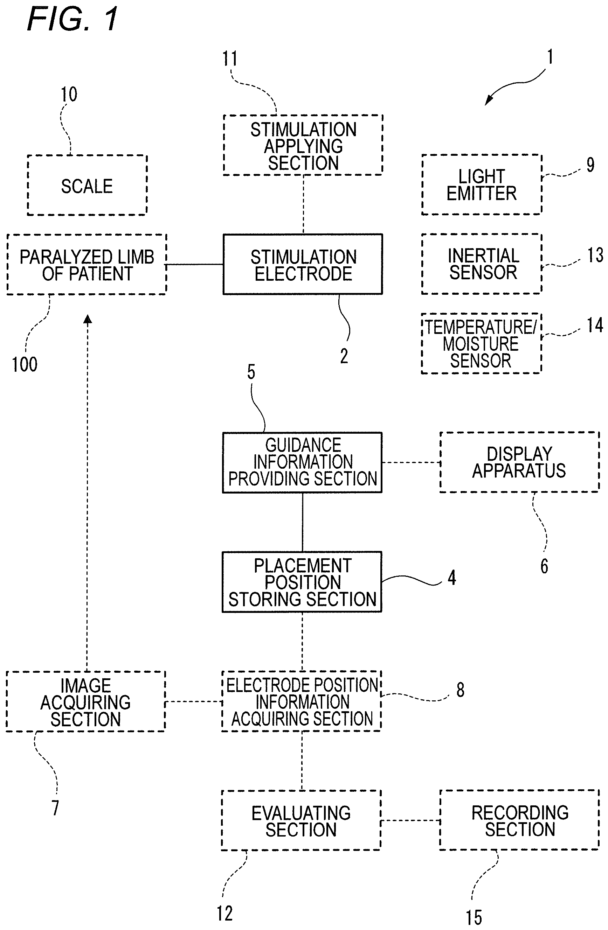

Hereinafter, embodiments will be described in detail with reference to the accompanying drawings. Fig. 1 is a functional block diagram illustrating a rehabilitation assistance system 1 (hereinafter, abbreviated as the assistance system 1) of a first embodiment. The assistance system 1 of the embodiment is configured so as to assist rehabilitation which is performed on a hemiplegia patient after, for example, stroke. The assistance system 1 includes at least one stimulation electrode 2.

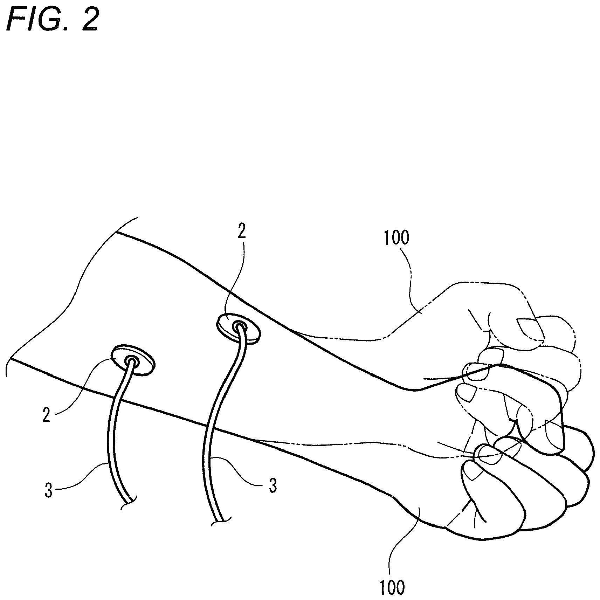

As illustrated in Fig. 2, the stimulation electrodes 2 are configured so as to be placed on a paralyzed limb 100 (an example of a part of the body) of the patient. For example, the stimulation electrodes 2 are used for, through electricity stimulation, causing the paralyzed limb 100 to repetitively operate, thereby expediting the recovery of the brain and the nerves. The stimulation electrodes 2 are connected to an electrical stimulation apparatus which is not shown, through cords 3. The electrical stimulation apparatus is configured so as to detect a command which instructs the paralyzed limb 100 to move, and which is issued by the brain of the patient, or by the assistance system 1, and energize the stimulation electrodes 2 based on the command. Electrical stimulation which is applied to the paralyzed limb 100 through the stimulation electrodes 2 causes the paralyzed limb 100 to move as indicated by the dash-dot-dot line in Fig. 2. Therefore, the patient can feel as if the paralyzed limb moves as intended. It is known that repetition of such stimulation expedites the recovery of the function of a paralyzed limb.

As illustrated in Fig. 1, the assistance system 1 includes a placement position storing section 4. The placement position storing section 4 stores placement position information indicative of positions where the stimulation electrodes 2 are to be placed. For example, the placement position information may be three-dimensional coordinates in a space including the paralyzed limb 100. In this case, the positions where the stimulation electrodes 2 are to be placed can be designated by specific three-dimensional coordinates. Examples of the placement position storing section 4 are a memory element, a large-capacity storage device (such as a hard disk drive), and a portable storage medium (a disk-type storage medium, a card-type storage medium, a USB memory, or the like).

As illustrated in Fig. 1, the assistance system includes a guidance information providing section 5. The guidance information providing section 5 is configured so as to, based on the placement position information stored in the placement position storing section 4, provide guidance information for guiding the stimulation electrodes 2 to the positions indicated by the placement position information, to the practitioner (an example of the user). The practitioner places the stimulation electrodes 2 on predetermined positions of the paralyzed limb 100 based on the guidance information provided by the guidance information providing section 5.

According to the configuration, in a plurality of opportunities, positions where the stimulation electrodes 2 are placed on the patient are made constant irrespective of the practitioner. The positions are determined as positions where an effect of rehabilitation is properly exerted. Therefore, the effectiveness of rehabilitation which is performed in a plurality of opportunities can be enhanced. Moreover, the placement positions of the stimulation electrodes 2 are always constant, and therefore the reliability of evaluation of rehabilitation which is performed in a plurality of opportunities can be enhanced.

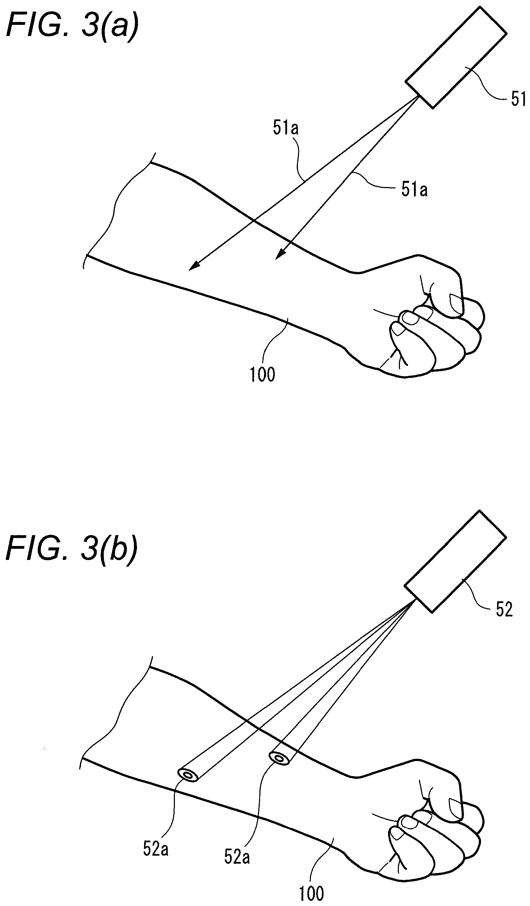

Fig. 3(a) illustrates a pointer 51 which is an example of the guidance information providing section 5. The pointer 51 includes a light source which can emit light beams 51a. Any kind of light source may be used as far as the light source can emit light beams having a certain level of directionality. The pointer 51 emits the light beams 51a (an example of the guidance information) indicating points on the paralyzed limb 100 which correspond to the three-dimensional coordinates stored in the placement position storing section 4. The points indicated by the light beams 51a are positions which are specified by the three-dimensional coordinates, and at which the stimulation electrodes 2 are to be placed. The practitioner places the stimulation electrodes 2 at the positions indicated by the light beams 51a.

According to the configuration, the placement positions of the stimulation electrodes 2 are optically indicated on the paralyzed limb 100 by the light beams 51a, and therefore the practitioner can correctly place the stimulation electrodes 2 at the predetermined positions while being guided by the light beams 51a. In a plurality of opportunities, therefore, the positions where the stimulation electrodes 2 are placed on the patient can be made constant irrespective of the practitioner.

Fig. 3(b) illustrates a projector 52 which is an example of the guidance information providing section 5. The projector 52 is configured so as to be able to project images 52a corresponding to the stimulation electrodes 2. The projector 52 projects the images 52a (an example of the guidance information) onto the points which are on the paralyzed limb 100, and which correspond to the three-dimensional coordinates stored in the placement position storing section 4. The points onto which the images 52a are projected are positions which are specified by the three-dimensional coordinates, and at which the stimulation electrodes 2 are to be placed.

According to the configuration, the placement positions of the stimulation electrodes 2 are optically indicated on the paralyzed limb 100 by the images 52a, and therefore the practitioner can correctly place the stimulation electrodes 2 at the predetermined positions while being guided by the images 52a. In a plurality of opportunities, therefore, the positions where the stimulation electrodes 2 are placed on the patient can be made constant irrespective of the practitioner.

As indicated by broken lines in Fig. 1, the assistance system 1 may have a configuration including a display apparatus 6. Examples of the display apparatus 6 are an installation-type display apparatus (such as a display apparatus or a monitor apparatus), and a portable display terminal (such as a smart phone, a tablet terminal, or a head-mounted display). The display apparatus 6 is communicably connected to the guidance information providing section 5. The guidance information providing section 5 causes the guidance information to be displayed on the display apparatus 6.

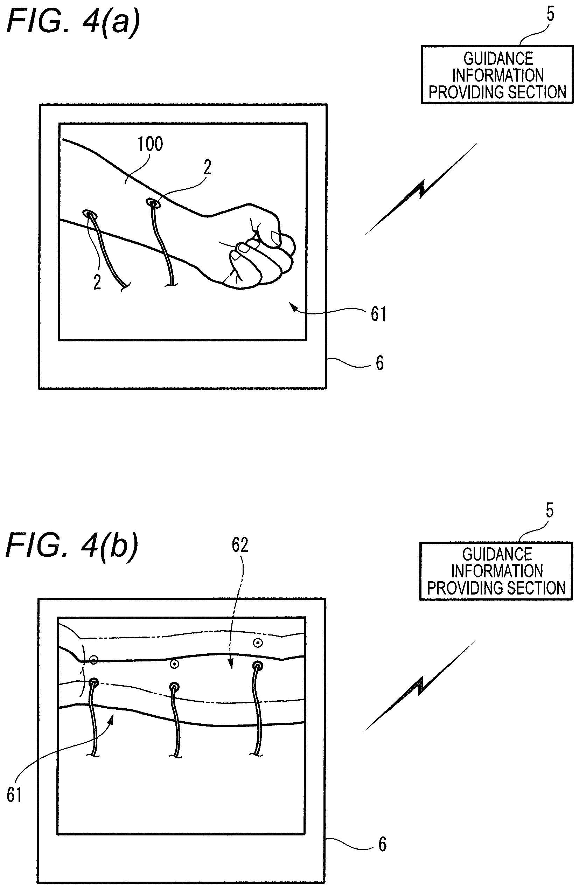

Fig. 4(a) illustrates an example in which an image 61 is displayed as guidance information on the display apparatus 6. The image 61 includes the paralyzed limb 100 of the patient in which the stimulation electrodes 2 are placed in rehabilitation that was performed in the past. Examples of the image 61 are an image which was taken by adequate imaging means, and a computer graphics image in which modeling is performed based on a taken image.

According to the configuration, the practitioner can correctly place the stimulation electrodes 2 at predetermined positions while checking the positions where the stimulation electrodes 2 was placed in past rehabilitation. In a plurality of opportunities, therefore, the positions where the stimulation electrodes 2 are placed on the patient can be made constant irrespective of the practitioner.

As illustrated in Fig. 4(b), an image 62 which is obtained by imaging the present paralyzed limb 100 of the patient may be displayed on the display apparatus 6. In this case, the guidance information providing section 5 causes the image 61 to be displayed in juxtaposition with the image 62 or superimposedly on the display apparatus 6. According to the configuration, the correctness of placement of the stimulation electrodes 2 is further improved. Alternatively, the rate of concordance between the image 61 and the image 62 may be detected, and an index corresponding to the rate of concordance may be displayed on the display apparatus 6. According to the configuration, the correctness of placement of the stimulation electrodes 2 is further improved, and moreover misidentification of patients or electrode placement portions can be prevented from occurring.

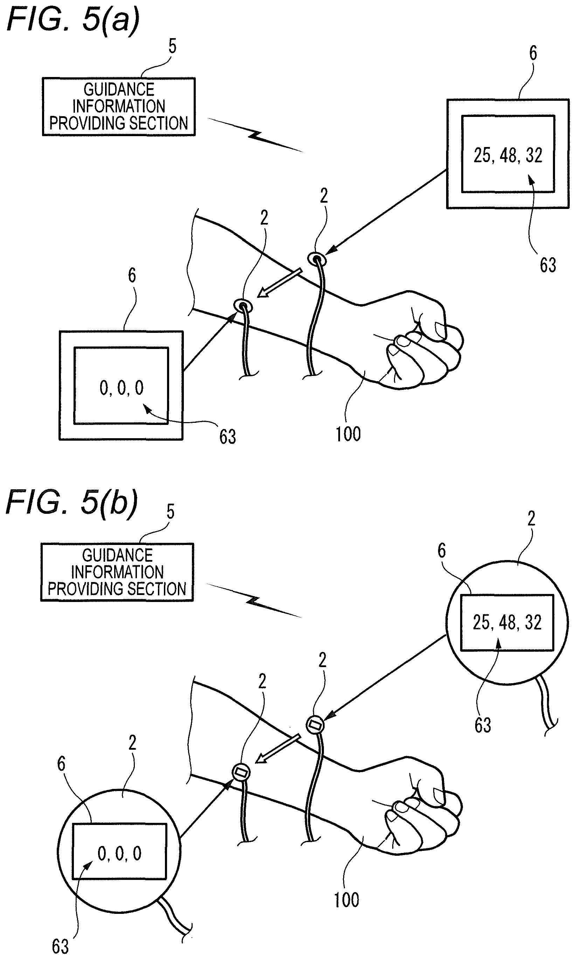

As illustrated in Fig. 5(a), a numerical value 63 indicating the distance to the position where the stimulation electrode 2 is to be placed may be displayed as guidance information on the display apparatus 6. In this case, the guidance information providing section 5 calculates the difference between the three-dimensional coordinates (the position where the stimulation electrode 2 is to be placed) indicated by the placement position information stored in the placement position storing section 4, and the three-dimensional coordinates indicating the current position of the stimulation electrode 2, and causes a result of the calculation on the display apparatus 6. The current position of the stimulation electrode 2 may be detected by adequate imaging means or a sensor. When the stimulation electrode 2 is placed at the predetermined position, the difference between the three-dimensional coordinates indicated by the placement position information, and the three-dimensional coordinates indicating the current position is zero. The practitioner can recognize that the stimulation electrode 2 is placed at the predetermined position, because of the fact that the numerical value 63 displayed on the display apparatus 6 is zero.

According to the configuration, the practitioner can correctly place the stimulation electrode 2 at the predetermined position while checking that the numerical value 63 approaches zero. In a plurality of opportunities, therefore, the position where the stimulation electrode 2 is placed on the patient can be made constant irrespective of the practitioner.

As illustrated in Fig. 5(b), the display apparatus 6 which displays the numerical value 63 indicating the distance to the position where the stimulation electrode 2 is to be placed may be disposed in a part of the stimulation electrode 2. According to the configuration, the practitioner can determine the placement of the stimulation electrode 2 while checking the numerical value 63 displayed on the display apparatus 6 disposed in the stimulation electrode 2. In a plurality of opportunities, therefore, the position where the stimulation electrode 2 is placed on the patient can be made constant irrespective of the practitioner.

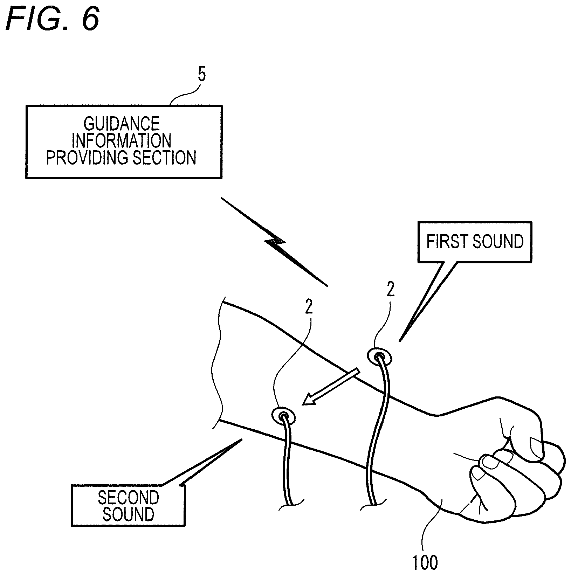

Fig. 6 illustrates an example in which a sound that changes in accordance with the distance to the position where the stimulation electrode 2 is to be placed is used as the guidance information. In this case, the guidance information providing section 5 calculates the difference between the three-dimensional coordinates (the position where the stimulation electrode 2 is to be placed) indicated by the placement position information stored in the placement position storing section 4, and the three-dimensional coordinates indicating the current position of the stimulation electrode 2, and outputs a sound corresponding to the value of the difference. The current position of the stimulation electrode 2 may be detected by adequate imaging means or a sensor.

In the example illustrated in Fig. 6, when the stimulation electrode 2 is at a position which is separated from the predetermined placement position, a first sound is output, and, when the stimulation electrode 2 is at the predetermined placement position, a second sound is output. An example of the difference between the first and second sounds is the volume of a sound. For example, when the stimulation electrode 2 is closer to the predetermined placement position, the larger volume of the output sound. Another example of the difference between the first and second sounds is the frequency (pitch) of a sound. For example, when the stimulation electrode 2 is closer to the predetermined placement position, the higher the output sound (frequency). Further examples of the difference between the first and second sounds are the interval of sounds which are repeatedly output, and the like. For example, as closer the stimulation electrode 2 to the predetermined placement position, the shorter interval of sounds which are repeatedly output. The first sound may be set as a silent sound, and the second sound may be set as a predetermined sound. In this case, only when the stimulation electrode 2 is placed at the predetermined position, the predetermined sound is output. The relationships of the volumes of sounds, levels of sounds (frequencies), and lengths of the intervals which are described above may be reversed.

According to the configuration, the practitioner can correctly place the stimulation electrode 2 at the predetermined position while being guided by the output sound. In a plurality of opportunities, therefore, the positions where the stimulation electrodes 2 are placed on the patient can be made constant irrespective of the practitioner.

Next, a configuration for acquiring the placement position information which is to be stored in the placement position storing section 4 will be described in detail. As indicated by broken lines in Fig. 1, the assistance system 1 may have a configuration including an image acquiring section 7 and an electrode position information acquiring section 8.

The image acquiring section 7 is configured so as to acquire an image of the paralyzed limb 100 of the patient. In order to know the three-dimensional shape of the paralyzed limb 100 of the patient, preferably, the image acquiring section 7 may be configured so as to be able to acquire depth information. The electrode position information acquiring section 8 is configured so as to acquire electrode position information indicating the position of the stimulation electrode 2 placed on the paralyzed limb 100, through the image acquired by the image acquiring section 7. The placement position storing section 4 is configured so as to be able to store the electrode position information acquired by the electrode position information acquiring section 8, as the above-described placement position information.

According to the configuration, the placement of the stimulation electrode 2 on the paralyzed limb 100 which is determined in rehabilitation performed at a certain timing is stored, and can be used in the next and subsequent rehabilitation processes. In a plurality of opportunities, therefore, the position where the stimulation electrode 2 is placed on the patient can be made constant irrespective of the practitioner. In order to acquire electrode position information through the image recognition technique, preferably, the stimulation electrode 2 may be colored with a color (blue, purple, pink, or the like) which can be clearly distinguished from the skin of the patient.

As indicated by broken lines in Fig. 1, the assistance system 1 may have a configuration including a light emitter 9. The light emitter 9 is placed adjacent to the stimulation electrode 2, and configured so as to emit light having a predetermined wavelength. The term "light having a predetermined wavelength" means that the light may be not only light in the visible range, but also light in the infrared range. The light emitter 9 may be disposed as a part of the stimulation electrode 2.

According to the configuration, the position of the stimulation electrode 2 is detected more easily by the image recognition technique performed by the image acquiring section 7. Preferably, the image acquiring section 7 has a high sensitivity to the wavelength of the light which is emitted by the light emitter 9. This enables the position of the stimulation electrode 2 to be detected more correctly, and the accuracy of the placement position information which is to be stored in the placement position storing section 4, to be improved. In a plurality of opportunities, therefore, the position where the stimulation electrode 2 is placed on the patient can be made constant irrespective of the practitioner.

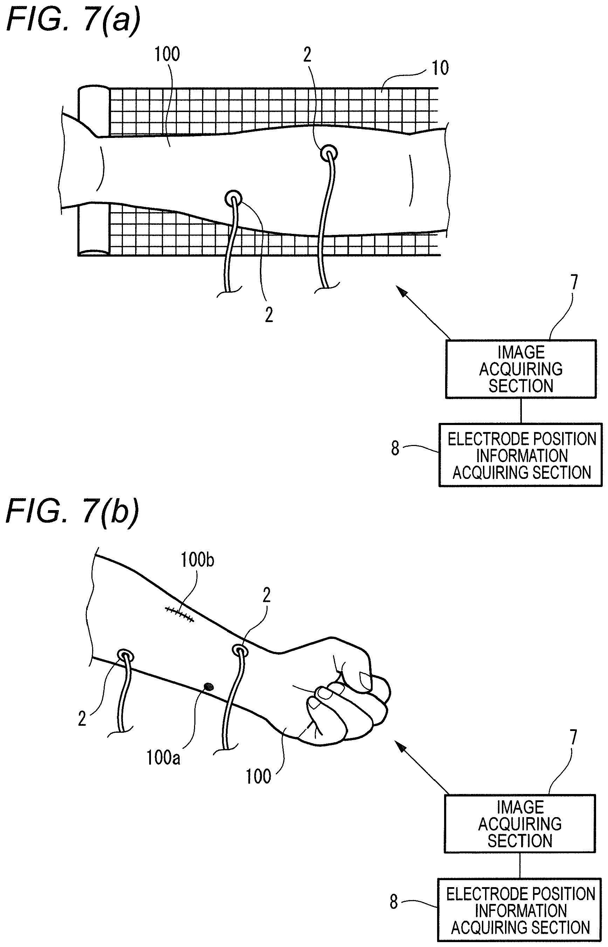

As indicated by broken lines in Fig. 1, the assistance system 1 may have a configuration including a scale 10. As illustrated in Fig. 7(a), the scale 10 is disposed adjacent to the paralyzed limb 100 of the patient, and has normalized scale marks. Here, the term "normalized" means that one unit (here, one scale mark) on the scale is corresponded to another unit such as 1 cm or 1 inch. In place of the marked scale, an article the size of which is normalized may be placed adjacent to the paralyzed limb 100 of the patient.

According to the configuration, the image acquiring section 7 performs image recognition with respect to the marks of the scale 10, and can correctly specify a position on the paralyzed limb 100 without using the distance between the image acquiring section 7 and the paralyzed limb 100, or the like. Consequently, the position of the stimulation electrode 2 is detected more correctly, and the accuracy of the placement position information which is to be stored in the placement position storing section 4 can be improved. In a plurality of opportunities, therefore, the position where the stimulation electrode 2 is placed on the patient can be made constant irrespective of the practitioner.

The image acquiring section 7 may be configured so as to extract feature points such as a lentigo 100a and a scar 100b on the paralyzed limb 100 of the patient such as illustrated in Fig. 7(b). In this case, the electrode position information acquiring section 8 may cause information indicating the feature points to be contained in the electrode position information. For example, the position of the stimulation electrode 2 may be specified based on the direction and distance relative to the lentigo 100a. The position of a blood vessel and the like in the paralyzed limb 100 may be extracted as a feature point.

According to the configuration, even when the scale 10 or the like is not used, the position of the stimulation electrode 2 can be specified by using the feature point on the part of the body of the patient. The feature point itself is not changed over a plurality of processes of rehabilitation, and hence the placement position of the stimulation electrode 2 can be specified with respect to the feature point also in the next and subsequent rehabilitation processes. In a plurality of opportunities, therefore, the position where the stimulation electrode 2 is placed on the patient can be made constant irrespective of the practitioner.

Next, a configuration for quantitatively evaluating an effect of rehabilitation will be described. As indicated by broken lines in Fig. 1, the assistance system 1 may have a configuration including a stimulation applying section 11 and an evaluating section 12.

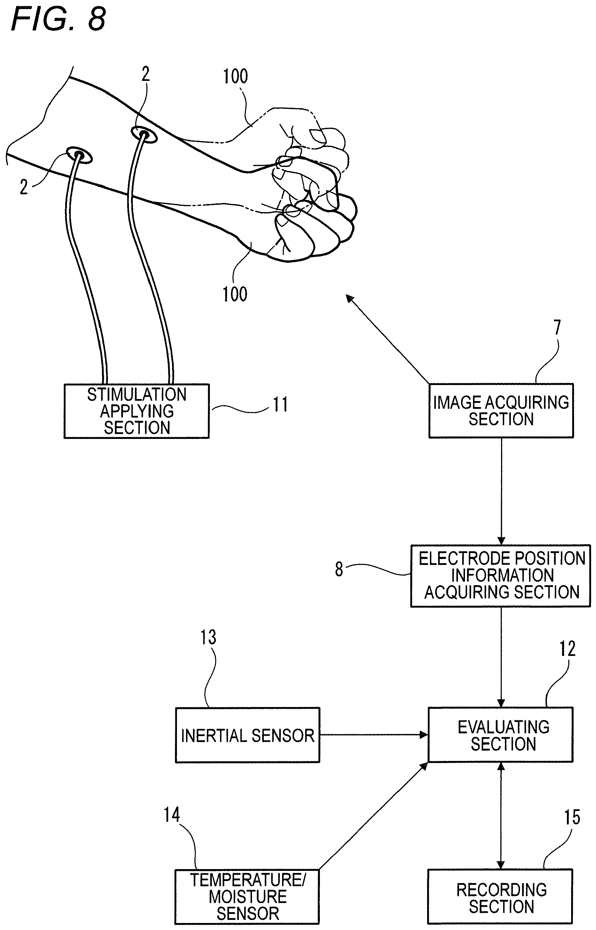

As illustrated in Fig. 8, the stimulation applying section 11 is configured so as to be able to apply electrical stimulation to the paralyzed limb 100 of the patient through the stimulation electrodes 2. In response to the electrical stimulation, the paralyzed limb 100 performs a reflex motion. The image acquiring section 7 acquires the manner where the paralyzed limb 100 performs a reflex motion, as image data. Based on the image acquired by the image acquiring section 7, the electrode position information acquiring section 8 acquires electrode position information of the stimulation electrodes 2 before and after the electrical stimulation.

The evaluating section 12 is configured so as to evaluate an effect of rehabilitation based on a change of the electrode position information, the change being caused by the electrical stimulation. Specifically, the electrode position information contains the three-dimensional position coordinates of the stimulation electrodes 2, as information, and therefore the direction and degree of the motion which is performed by the paralyzed limb 100 in response to certain electrical stimulation can be quantitatively known based on the displacements of the stimulation electrodes 2.

According to the configuration, a reaction of the paralyzed limb 100 to electrical stimulation, i.e., the effect of rehabilitation can be quantitatively evaluated without depending on visual observation by an observer. Electrical stimulation is performed through the stimulation electrodes 2 which are correctly placed at the constant positions as described above. In a plurality of opportunities, therefore, correctness can be ensured in both an input of stimulation and a reaction to the stimulation.

As indicated by broken lines in Fig. 1, the assistance system 1 may have a configuration including an inertial sensor 13. The inertial sensor 13 is placed adjacent to the stimulation electrode 2. The inertial sensor 13 may be disposed as a part of the stimulation electrode 2. The inertial sensor 13 is configured so as to output a signal corresponding to the displacement (the velocity, acceleration, and attitude change) of the stimulation electrode 2 due to electrical stimulation. As illustrated in Fig. 8, the evaluating section 12 is configured so as to, in addition to a change of the electrode position information due to electrical stimulation, evaluate the effect of rehabilitation based on the output of the inertial sensor 13.

According to the configuration, it is possible to acquire not only information of the displacement of the stimulation electrode 2 in the three-dimensional space, but also that of the displacement velocity and attitude change of the stimulation electrode 2 in the three-dimensional space. Therefore, the effect of rehabilitation can be evaluated in more detail.

As indicated by broken lines in Fig. 1, the assistance system 1 may have a configuration including a temperature/moisture sensor 14. The temperature/moisture sensor 14 is placed adjacent to the stimulation electrode 2. The temperature/moisture sensor 14 may be disposed as a part of the stimulation electrode 2. The temperature/moisture sensor 14 is configured so as to output a signal corresponding to at least one of the temperature and moisture of the place where the sensor itself is placed. As illustrated in Fig. 8, the evaluating section 12 is configured so as to evaluate an effect of rehabilitation based on the output from the temperature/moisture sensor 14 in addition to a change of the electrode position information due to electrical stimulation, or in addition to or in place of the output of the inertial sensor 13.

It is known that, in accordance with the recovery of the function of a paralyzed portion, the temperature and moisture of the portion are raised. According to the above-described configuration, it is possible to acquire information of the degree of recovery based on not only displacement of the stimulation electrode 2 in the three-dimensional space, but also at least one of the temperature and moisture of the paralyzed limb 100. Therefore, the effect of rehabilitation can be evaluated in more detail.

As indicated by broken lines in Fig. 1, the assistance system 1 may have a configuration including a recording section 15. The recording section 15 is configured so as to record the electrode position information acquired by the electrode position information acquiring section 8, while associating the electrode position information with at least the timing when the electrode position information is acquired, and patient identification information (the name, the patient number, or the like). Examples of other information to be recorded in the recording section 15 are the name of disease, conditions of treatment, contents of treatment, and the like.

Examples of the recording section 15 are a large-capacity storage device (such as a hard disk drive), and a portable storage medium (a disk-type storage medium, a card-type storage medium, a USB memory, or the like). The installation site of the recording section 15 is not particularly limited as far as the section can communicate with the electrode position information acquiring section 8 in a wired or wireless manner. The recording section may constitute a stand-alone system together with the electrode position information acquiring section 8, or be connected with the electrode position information acquiring section 8 via a LAN or a WAN.

As illustrated in Fig. 8, the evaluating section 12 is configured so as to compare the electrode position information at a plurality of timings with one another to evaluate the effect of rehabilitation. In the comparison, as at least part of the the electrode position information at a plurality of timings, information stored in the recording section 15 is referred. In the case where the same electrical stimulation is applied through the stimulation electrode 2 which is placed at the same place based on the above description, for example, the reaction in the previous rehabilitation, and that in the present rehabilitation are compared to each other, whereby the progress of rehabilitation can be quantitatively evaluated. Alternatively, images at a plurality of timings which were taken by the image acquiring section 7 under the same imaging conditions are previously recorded in the recording section 15, and the correlation factor of the plural images is evaluated. Also in the process, the progress of rehabilitation can be quantitatively evaluated. The evaluation of the correlation factor of images may be performed not only in a mode where the predetermined state (for example, at the timing when the motion is ended) of the paralyzed limb 100 is evaluated, but also in that where the motion trajectory, the velocity, and the like are evaluated based on acquired images.

According to the configuration, a change of a reaction of the paralyzed limb 100 to the electrical stimulation, i.e., the progress of rehabilitation can be quantitatively evaluated without depending on visual observation by an observer. Electrical stimulation is performed through the stimulation electrodes 2 which are correctly placed at the constant positions as described above. In a plurality of opportunities, therefore, correctness can be ensured in both an input of stimulation and a reaction to the stimulation.

In the embodiment, the functions of at least the electrode position information acquiring section 8 and the evaluating section 12 are realized from software executed by a cooperation of a processor and memory which are communicably connected to each other. Examples of the processor are a CPU and an MPU. Examples of the memory are a RAM and a ROM. However, the function of at least one of the electrode position information acquiring section 8 and the evaluating section 12 may be realized by hardware such as circuit devices, or a combination of hardware and software.

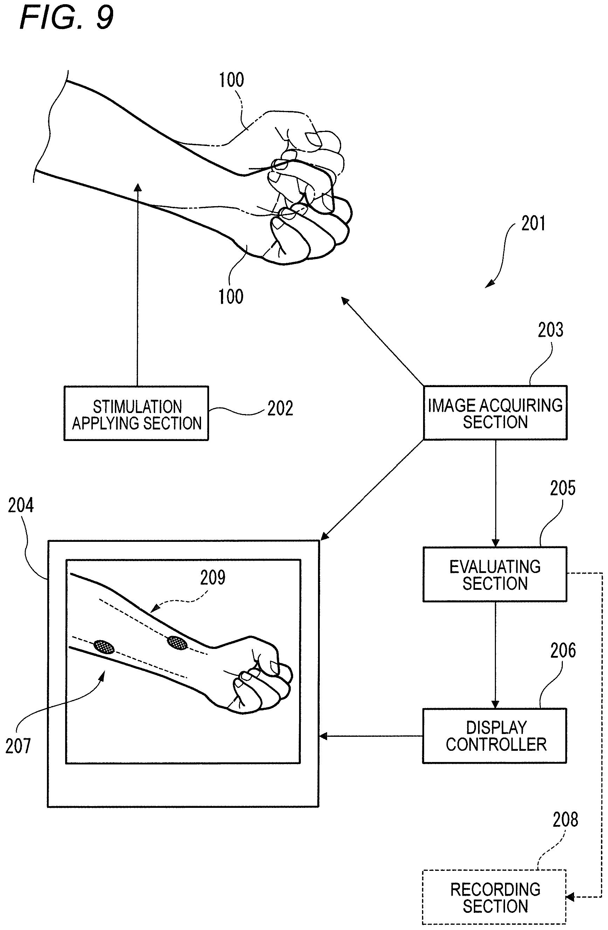

Fig. 9 is a functional block diagram illustrating a rehabilitation assistance system 201 (hereinafter, abbreviated as the assistance system 201) of a second embodiment. The assistance system 201 of the embodiment is configured so as to assist a work of determining an adequate placement position of a stimulation electrode. The assistance system 201 includes a stimulation applying section 202, an image acquiring section 203, a displaying section 204, an evaluating section 205, and a display controller 206.

The stimulation applying section 202 is configured so as to be able to apply electrical stimulation to the paralyzed limb 100 (an example of a part of the body of the patient) of the patient. In response to the electrical stimulation, the paralyzed limb 100 performs a reflex motion. The image acquiring section 203 acquires the manner where the paralyzed limb 100 performs a reflex motion, as image data. The displaying section 204 is configured so as to display the image acquired by the image acquiring section 203.

The evaluating section 205 is configured so as to evaluate the degree of a reaction of the paralyzed limb 100 to the electrical stimulation applied by the stimulation applying section 202, based on the image acquired by the image acquiring section 203. Specifically, the rate of concordance between the image of the paralyzed limb 100 before the application of stimulation, and that of the paralyzed limb 100 reacted to the stimulation is acquired, and it is evaluated that, the lower the rate of concordance, the larger reaction. Alternatively, an adequate marker is attached to the paralyzed limb 100, the amount of displacement of the marker due to the electrical stimulation is image recognized, and the degree of the reaction is evaluated. When electrical stimulation is applied by the stimulation applying section 202 to a plurality of places of the paralyzed limb 100, and the degrees of reactions of the paralyzed limb 100 to the respective stimulation applications are evaluated, thereby obtaining a plurality of sets in which the position where the electrical stimulation is applied is paired with the degree of reaction.

The display controller 206 is configured so as to cause a map 207 indicating relationships between the position where the electrical stimulation is applied by the stimulation applying section 202, and the degree of the reaction of the paralyzed limb 100 which is evaluated by the evaluating section 205, to be displayed on the displaying section 204. In the example illustrated in Fig. 9, stimulation application places where a larger reaction was obtained are displayed superimposedly on the image acquired by the image acquiring section 203, on the displaying section 204. The map 207 indicates positions where stimulation electrodes are to be placed in rehabilitation. The practitioner determines placement positions of stimulation electrodes based on the map 207.

The manner of displaying the map 207 is not limited to the example illustrated in Fig. 9. For example, contour lines corresponding to reaction degrees may be displayed superimposedly on the image acquired by the image acquiring section 203. The map displayed on the displaying section 204 is not required to be displayed superimposedly on the image acquired by the image acquiring section 203. The map may be provided as a computer graphics image which is produced by performing modeling based on the image of the paralyzed limb 100 that is taken by the image acquiring section 203.

As indicated by broken lines in Fig. 9, the assistance system 201 may have a configuration including a recording section 208. The recording section 208 is configured so as to record the result of the evaluation performed by the evaluating section 205, while associating the result with at least the timing when the evaluation is performed, and patient identification information (the name, the patient number, or the like). Examples of other information to be recorded in the recording section 208 are the name of disease, conditions of treatment, contents of treatment, and the like.

Examples of the recording section 208 are a large-capacity storage device (such as a hard disk drive), and a portable storage medium (a disk-type storage medium, a card-type storage medium, a USB memory, or the like). The installation site of the recording section 15 is not particularly limited as far as the section can communicate with the evaluating section 205 in a wired or wireless manner. The recording section may constitute a stand-alone system together with the evaluating section 205, or be connected with the evaluating section 205 via a LAN or a WAN. Alternatively, the recording section 208 may be the placement position storing section 4 which has been described with reference to the first embodiment.

According to the configuration, irrespective of an evaluator, the placement position of the stimulation electrode can be determined based on a quantitative evaluation. When the stimulation electrode is placed at the placement position which is determined as described above, the position where the stimulation electrode is placed on the patient can be made constant irrespective of the practitioner in a plurality of opportunities.

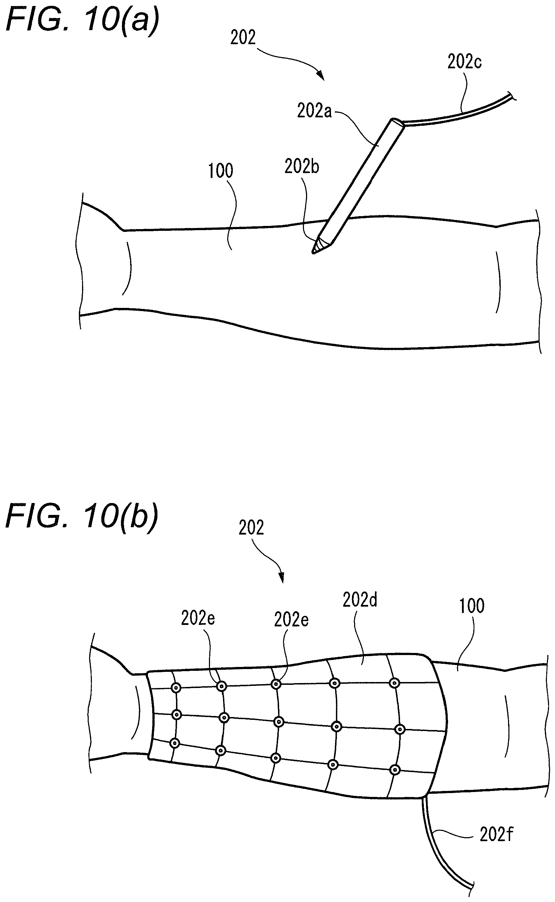

Fig. 10(a) illustrates an example of the stimulation applying section 202. In the stimulation applying section 202 of the example, a conducting portion 202b is disposed in the tip end of a rod member 202a. In accordance with a signal which is input through a signal wire 202c, electrical stimulation is applied from the conducting portion 202b to the paralyzed limb 100.

According to the configuration, without conducting a work of attaching/detaching the stimulation electrode to and from the paralyzed limb 100, the application of electrical stimulation to a plurality of arbitrary places on the paralyzed limb 100 can be efficiently performed. Therefore, a quantitative evaluation for determining the placement position of the stimulation electrode can be efficiently executed.

Fig. 10(b) illustrates another example of the stimulation applying section 202. The stimulation applying section 202 of the example includes a sheet member 202d which is to be attached to the paralyzed limb 100. A plurality of conducting portions 202e are arranged on the sheet member 202d. In accordance with a signal which is input through a signal wire 202f, electrical stimulation is applied sequentially from the conducting portions 202e to the paralyzed limb 100.

According to the configuration, when the sheet member 202d is once attached to the paralyzed limb 100, the application of electrical stimulation to a plurality of places on the paralyzed limb 100 can be efficiently performed. Particularly, the positional relationships among the plurality of conducting portions 202e are fixed, and hence it is possible to eliminate a situation where the stimulation position is differentiated depending on the practitioner. Therefore, a quantitative evaluation for determining the placement position of the stimulation electrode can be efficiently executed irrespective of the practitioner.

As indicated by the broken lines in Fig. 9, the display controller 206 may have a configuration where the controller causes an image 209 indicating at least one of muscle and nerve which are included in the paralyzed limb 100, to be displayed on the displaying section 204. The image 209 is a computer graphics image which is previously produced based on anatomical knowledge.

According to the configuration, the application position of stimulation performed by the stimulation applying section 202 can be determined by reference to the image 209. Therefore, application of electrical stimulation to a plurality of places on the paralyzed limb 100 can be efficiently performed.

In the embodiment, the functions of at least the evaluating section 205 and the display controller 206 are realized from software executed by a cooperation of a processor and memory which are communicably connected to each other. Examples of the processor are a CPU and an MPU. Examples of the memory are a RAM and a ROM. However, the function of at least one of the evaluating section 205 and the display controller 206 may be realized by hardware such as circuit devices, or a combination of hardware and software.

The above-described embodiments are examples for facilitating understanding of the invention, and do not limit the invention. The invention may be changed or improved without departing from the spirit of the invention. It is obvious that equivalents are included within the scope of the invention.

The disclosure of Japanese Patent Application No. 2014-115858 filed June 4, 2014 is incorporated in and constituting part of the description of the present application.

## Claims

1. A rehabilitation assistance system comprising:
a stimulation electrode which is to be placed on a part of a body of a patient;
a placement position storing section which stores placement position information indicative of a position where the stimulation electrode is to be placed; and
a guidance information providing section which provides guidance information for guiding the stimulation electrode to the position based on the placement position information, to a user.

2. The rehabilitation assistance system according to claim 1, wherein
the guidance information providing section optically indicates the guidance information on the part of the body of the patient.

3. The rehabilitation assistance system according to claim 1 or 2, further comprising
a display apparatus, wherein
the guidance information providing section causes the guidance information to be displayed on the display apparatus.

4. The rehabilitation assistance system according to claim 3, wherein
the guidance information contains an image of the part of the body of the patient, which is acquired in a past.

5. The rehabilitation assistance system according to claim 3, wherein
the guidance information is a numerical value indicating a distance to the placement position.

6. The rehabilitation assistance system according to claim 1, wherein
the guidance information is a sound which changes in accordance with a distance to the placement position.

7. The rehabilitation assistance system according to any one of claims 1 to 6, further comprising:
an image acquiring section which acquires an image of the part of the body of the patient; and
an electrode position information acquiring section which, from the image, acquires electrode position information indicative of a position where the stimulation electrode is placed on the patient, wherein
the placement position storing section stores the stimulation electrode position information as the placement position information.

8. The rehabilitation assistance system according to claim 7, further comprising
a light emitter which is placed adjacent to the stimulation electrode, and which emits light having a predetermined wavelength.

9. The rehabilitation assistance system according to claim 7 or 8, further comprising
a normalized scale which is to be placed adjacent to the part of the body of the patient.

10. The rehabilitation assistance system according to any one of claims 7 to 9, wherein
the stimulation electrode position information contains information indicative of a feature point included in the part of the body of the patient.

11. The rehabilitation assistance system according to any one of claims 7 to 10, further comprising:
a stimulation applying section which applies electrical stimulation to the part of the body of the patient through the stimulation electrode; and
an evaluating section which evaluates an effect of rehabilitation based on a change of the stimulation electrode position information, which is caused by the electrical stimulation.

12. The rehabilitation assistance system according to claim 11, further comprising
an inertial sensor which is placed adjacent to the stimulation electrode, wherein
the evaluating section evaluates the effect of rehabilitation based on an output of the inertial sensor.

13. The rehabilitation assistance system according to claim 11 or 12, further comprising
at least one of a temperature sensor and a moisture sensor which are placed adjacent to the stimulation electrode, wherein
the evaluating section evaluates the effect of rehabilitation based on an output of the at least one of the temperature sensor and the moisture sensor.

14. The rehabilitation assistance system according to any one of claims 11 to 13, further comprising
a recording section which records the stimulation electrode position information while associating the stimulation electrode position information with at least a timing when the electrode position information is acquired, and patient identification information, wherein
the evaluating section compares the stimulation electrode position information at a plurality of timings with one another to evaluate the effect of rehabilitation.

15. A rehabilitation assistance system comprising:
a stimulation applying section which applies electrical stimulation to a part of a body of a patient;
an image acquiring section which acquires an image of the part of the body of the patient;
an evaluating section which evaluates a degree of a reaction of the part of the body of the patient to the electrical stimulation, based on the image;
a displaying section which displays the image; and
a display controller which causes a map indicating a relationship between a position where the electrical stimulation is applied, and the degree of the reaction, to be displayed on the displaying section.

16. The rehabilitation assistance system according to claim 15, wherein
the stimulation applying section includes a conducting portion which is disposed in a tip end portion of a rod member.

17. The rehabilitation assistance system according to claim 15, wherein
the stimulation applying section includes a plurality of conducting portions which are arranged on a sheet member to be attached to the part of the body of the patient.

18. The rehabilitation assistance system according to any one of claims 15 to 17, wherein
the display controller causes an image indicating a position of at least one of muscle and nerve which are included in the part of the body of the patient, to be displayed on the displaying section.
